# EUROPEAN PATENT APPLICATION

(11) **EP 4 678 110 A1**
(43) Date of publication of application: **14.01.2026**
(21) Application number: 24767389.0
(22) Date of filing: 05.03.2024
(51) Int. Cl.: A61B 6/03, A61B 6/00

(54) **MEDICAL IMAGE GENERATION APPARATUS AND METHOD**

(30) Priority: 06.03.2023 KR 20230029052
(71) Applicant: Vatech Co., Ltd., Hwaseong-si, Gyeonggi-do 18449 (KR); Vatech Ewoo Holdings Co., Ltd., Hwaseong-si, Gyeonggi-do, 18449 (KR)
(72) Inventor: LIM, Sung Hwan, Hwaseong-si Gyeonggi-do 18449 (KR); PARK, Chul Kyu, Hwaseong-si Gyeonggi-do 18449 (KR); CHOI, Sung Il, Hwaseong-si Gyeonggi-do 18449 (KR)
(74) Representative: Lorenz & Kollegen
(86) International application number: PCT/KR2024/002813
(87) International publication number: WO 2024/186101

(57) **Abstract**

The present invention relates to a medical image generation apparatus, and the medical image generation apparatus may comprise: a projection data obtainment unit that obtains projection data for a field of view; a first medical image generation unit that generates, by using the projection data, a first medical image of a first resolution for the field of view; a region-of-interest selection unit that receives, on the basis of the first medical image, a selection of a region of interest, which is a part of the field of view; and a second medical image generation unit that generates, by using the projection data, a second medical image of a second resolution for the region of interest, the second resolution being higher than the first resolution.

## Description

### Technical Field

The present disclosure relates to a medical image generation apparatus and a medical image generation method.

### Background Art

Generally, a three-dimensional Computed Tomography (CT) image from various directions may be acquired by acquiring projection data of a Field of View from various direction by using X-rays and then by reconstructing the acquired projection data into a three-dimensional image.

Meanwhile, the time required to reconstruct the projection data from various directions into the three-dimensional image varies according to the size of the voxel that is a basic unit of the three-dimensional image, the size of the FOV, the number of projection data used for reconstruction, the performance of the hardware used for reconstruction, a reconstruction algorithm used for reconstruction, and so on. Since the time required for reconstruction has a trade-off relationship with a resolution of the three-dimensional image, it is difficult to reduce the time required for reconstruction while a high resolution of the three-dimensional image is maintained.

In addition, in a conventional technology, even when a user intends to first check only a part of a region in the three-dimensional image, the entire region of the three-dimensional image is required to be reconstructed, so that there is a disadvantage that the time required to check the part of the region is the same as the time required to check the entire region.

The technology that serves as a background of the present disclosure is disclosed in Korean Patent Application Publication No. 10-2015-0080820.

### Disclosure

### Technical Problem

Accordingly, the present disclosure has been made keeping in mind the above problems occurring in the related art, and an objective of the present disclosure is to provide a medical image generation apparatus and a medical image generation method, the apparatus and the method being configured such that a three-dimensional medical image of the entire Field of View (FOV) is generated at a relatively low resolution and a three-dimensional medical image of a region of interest selected by a user input is generated at a high resolution, thereby being capable of displaying the region of interest rapidly.

However, the problem to be solved by the present disclosure is not limited to the above-described problem, and there may be other problems to be solved by the present disclosure.

### Technical Solution

As a technical mechanism for realizing the technical objective of the present disclosure, according to an aspect of the present disclosure, there is provided a medical image generation apparatus including: a projection data acquisition unit configured to acquire projection data of a field of view; a first medical image generation unit configured to generate a first medical image of the field of view at a first resolution by using the projection data; a region-of-interest selection unit configured to receive a selection of a region of interest that is a part of the field of view on the basis of the first medical image; and a second medical image generation unit configured to generate a second medical image of the region of interest at a second resolution by using the projection data, the second resolution being higher than the first resolution.

As a technical mechanism for realizing the technical objective of the present disclosure, according to an aspect of the present disclosure, there is provided a medical image generation method including: (a) acquiring projection data corresponding to a field of view; (b) generating a first medical image of the field of view at a first resolution by using the projection data; (c) receiving a selection of a region of interest that is a part of the field of view on the basis of the first medical image; and (d) generating a second medical image of the region of interest at a second resolution by using the projection data, the second resolution being higher than the first resolution.

The above-described technical solutions are set forth to illustrate only and should not be construed as intended to limit the present disclosure. In addition to the exemplary embodiments described above, additional embodiments may exist in the drawings and detailed description of the present disclosure.

### Advantageous Effects

According to the technical solution described above, since the three-dimensional medical image of the entire field of view is generated at the relatively low resolution and the three-dimensional medical image of the region of interest selected by the user input is generated at the high resolution, there is an effect that the region of interest is capable of being displayed with the high resolution rapidly.

However, the effects capable of being acquired from the present disclosure are not limited to the effects described above, and other effects may also exist.

### Description of Drawings

FIG. 1 is a block diagram schematically illustrating a medical image generation apparatus according to an embodiment of the present disclosure.
FIG. 2(a) and FIG. 2(b) show an example of a screen in which regions of interest provided by the medical image generation apparatus according to an embodiment of the present disclosure are selected.
FIG. 3(a), FIG. 3(b), and FIG. 3(c) show a difference in resolution according to voxel sizes of a first medical image and a second medical image provided by the medical image generation apparatus according to an embodiment of the present disclosure. FIG. 4a and FIG. 4b are views illustrating the first medical image expressed in three dimensions and the second medical image expressed in three dimensions that are provided by the medical image generation apparatus according to an embodiment of the present disclosure.
FIG. 5 is an operation flowchart illustrating a medical image generation method according to an embodiment of the present disclosure.

### Mode for Invention

Hereinafter, embodiments of the present disclosure will be described in detail such that those skilled in the art to which the present disclosure belongs may easily implement the present disclosure with reference to the accompanying drawings. However, the present disclosure may be implemented in many different forms and is not limited to the embodiments to be described herein. In addition, in order to clearly describe the present disclosure with reference to the drawings, parts irrelevant to the description are omitted, and similar reference numerals denote similar parts throughout the specification.

Throughout the present specification, when a part is described to be "connected" to another part, this includes not only a case where the part is "directly connected" thereto, but also a case where the part is "indirectly connected" or "electrically connected" thereto with another element therebetween.

Throughout the present specification, when a member is referred to as being "on", "on an upper portion of", "on an upper end of", "under", "on a lower portion of", or "on a lower end of" another member, this may include not only the case where a member is in contact with another member, but also the case where another member exists between two members.

Throughout the present specification, when a part is referred to as "including" an element, it means that the part may include other elements as well without excluding the other elements unless specifically stated otherwise.

The present disclosure relates to a medical image generation apparatus and a medical image generation method.

Throughout the present specification, the term "subject" may refer to a person who will receive a diagnosis and an observation of teeth (intraoral cavity) by a medical image generation apparatus 100 according to an embodiment of the present disclosure, i.e., a patient. Furthermore, the term "user" may refer to a person performing the diagnosis and the observation of the subject's teeth (intraoral cavity) by using the medical image generation apparatus 100 according to an embodiment of the present disclosure, i.e., a medical personnel (a doctor, a nurse, and so on), but the terms are not limited thereto.

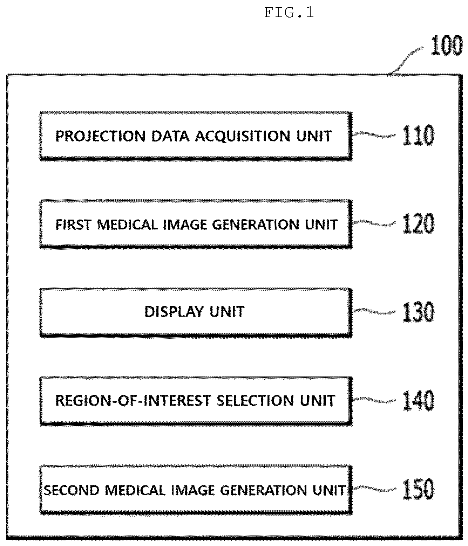

FIG. 1 is a block diagram schematically illustrating a medical image generation apparatus according to an embodiment of the present disclosure.

Referring to FIG. 1, the medical image generation apparatus 100 may include a projection data acquisition unit 110, a first medical image generation unit 120, a display unit 130, a region-of-interest selection unit 140, and a second medical image generation unit 150.

In addition, although not illustrated, the medical image generation apparatus 100 may further include an input unit, a control unit, and a storage unit.

The input unit may be configured to receive a user input including an input for a selection of a region of interest. For example, the input unit may include a mouse, a keyboard of a computer, a keypad, a touchpad, and so on, but is not limited thereto. Furthermore, the input unit may include a Graphic User Interface (GUI) that can be controlled by the input unit.

In addition, the control unit may refer to a central processing unit configured to control an overall operation of the medical image generation apparatus 100 according to an embodiment of the present disclosure. For example, the control unit may be implemented by using at least one selected from Application Specific Integrated Circuits (ASICs), Digital Signal Processors (DSPs), Digital Signal Processing Devices (DSPDs), Programmable Logic Devices (PLDs), Field-Programmable Gate Arrays (FPGAs), processors, controllers, micro-controllers, and microprocessors, or may be implemented as a firmware module or a software module capable of being executed on a hardware platform described above. At this time, the firmware module or the software module may be implemented by one or more software applications written in an appropriate program language.

In addition, the storage unit may be configured to store various images, a variable value, initial projection data, a first medical image, a second medical image, and so on for controlling the operation of the medical image generation apparatus 100 according to an embodiment of the present disclosure. For example, the storage unit may be implemented as one storage medium of a flash memory type, a hard disk type, a MultiMedia Card (MMC) type, a card type memory (for example, a Secure Digital (SD) card or an eXtreme Digital (XD) card), a Random Access Memory (RAM), a Static Random Access Memory (SRAM), a Read-Only Memory (ROM), an Electrically Erasable Programmable Read-Only Memory (EEPROM), a Programmable Read-Only Memory (PROM), a magnetic memory, a magnetic disk, and an optical disk, but is not limited thereto.

Here, the display unit 130 may be configured to output the first medical image, a cross-sectional image, the second medical image, and so on generated by the medical image generation apparatus 100 according to an embodiment of the present disclosure. For example, the display unit 130 may refer to at least one selected from a smartphone, a smart pad, a tablet PC, a wearable device, all kinds of wireless communication devices such as a Personal Communication System (PCS), a Global System for Mobile communication (GSM), a Personal Digital Cellular (PDC), a Personal Handyphone System (PHS), a Personal Digital Assistant (PDA), an International Mobile Telecommunication (IMT)-2000, a Code Division Multiple Access (CDMA)-2000, a Wideband Code Division Multiple Access (W-CDMA), a Wireless Broadband Internet (WIBRO) terminal, a fixed-type terminal such as a desktop computer and a smart TV, and various types of display devices capable of displaying an image, such as an LCD display, an LED display, an AMOLED display, a CRT display, and so on, but is not limited thereto.

At this time, according to an embodiment of the present disclosure, the projection data acquisition unit 110, the first medical image generation unit 120, the region-of-interest selection unit 140, and the second medical image generation unit 150 may be mounted on at least one selected from the control unit and the storage unit.

In addition, the input unit, the control unit, the storage unit, and the display unit 130 may be linked with each other through a network. For example, the network may include a 3rd Generation Partnership Project (3GPP) network, a Long Term Evolution (LTE) network, a 5G network, a World Interoperability for Microwave Access (WIMAX) network, a wired/wireless Internet, a Local Area Network (LAN), a Wireless Local Area Network (Wireless LAN), a Wide Area Network (WAN), a Personal Area Network (PAN), a Bluetooth network, a Wi-Fi network, a Near Field Communication (NFC) network, a satellite broadcasting network, an analog broadcasting network, a Digital Multimedia Broadcasting (DMB) network, and so on, but is not limited thereto.

The medical image generation apparatus 100 according to an embodiment of the present disclosure does not use projection data so as to reconstruct a high resolution three-dimensional medical image of the entire Field of View (FOV), but primarily reconstructs a low resolution three-dimensional medical image of the entire FOV and then secondly reconstructs only a part (a partial region that the user intends to check first, i.e., a region of interest) of the entire FOV in a high resolution three-dimensional medical image, so that only a small region compared to the entire FOV is reconstructed in a high resolution instead of wasting time reconstructing the entire FOV, thereby being capable of providing the high resolution three-dimensional medical image for the part that requires priority confirmation to the user in a shorter time compared to the time required to reconstruct the entire FOV.

Prior to the detailed description of the medical image generation apparatus 100 according to an embodiment of the present disclosure, the first medical image and the second medical image disclosed herein may, for example, be Computed Tomography (CT) images configured in units of voxels, but are not limited thereto, and may include three-dimensional medical images that represent the FOV in three dimensions. In the following, for convenience, the first medical image and the second medical image are described as CT images.

In addition, in the three-dimensional image, the resolution may correspond to the number of voxels included in a unit volume, and may be inversely proportional to the voxel size. In other words, the resolution may be lower when the number of voxels contained in the unit volume is smaller and the size of each individual voxel is larger.

Hereinafter, each component of the medical image generation apparatus 100 according to an embodiment of the present disclosure will be described in detail.

According to an embodiment of the present disclosure, the projection data acquisition unit 110 may acquire projection data of the FOV in which a subject exists. At this time, the projection data may include projection data from various directions for the subject. In addition, the subject may, for example, refer to the head including the maxillofacial region, but is not limited thereto.

At this time, the projection data acquisition unit 110 may be configured as a separate medical imaging apparatus separated from the medical image generation apparatus 100 according to an embodiment of the present disclosure, and may be a CT imaging apparatus as an example. In this situation, the projection data acquired by the projection data acquisition unit 110 may be transmitted to the medical image generation apparatus 100 according to an embodiment of the present disclosure in a wired manner or a wireless manner, and may be stored in the storage unit.

In a conventional technology, since computational load when a CT image is generated and reconstructed by using acquired projection data is required to be considered in acquiring the projection data, the projection data is acquired with a somewhat lower resolution so that the computational load is not excessively generated. However, the medical image generation apparatus 100 according to an embodiment of the present disclosure is configured to reconstruct a high resolution CT image only for a region of interest that is a part of the entire FOV, so that the time and the computational load that are required are less compared to that of the reconstruction of the entire FOV, thereby being capable of acquiring initial projection data with a higher resolution compared to the resolution in the conventional technology.

In other words, the medical image generation apparatus 100 has less need to consider an excessive time and an excessive computational load required for reconstruction compared to the conventional technology even when the initial projection data with the high resolution is acquired, so that there is no need to worry about a problem such as overload.

According to an embodiment of the present disclosure, the first medical image generation unit 120 may generate the first medical image of the FOV at a first resolution by using the projection data.

At this time, the first resolution may refer to a relatively low resolution, and the first medical image may be an image generated (reconstructed) with the first resolution with a relatively large voxel size through binning of the projection data, i.e., the low resolution.

Specifically, the first medical image generation unit 120 may be configured to generate the first medical image of the entire FOV by using the projection data, and may be configured to reconstruct the first medical image at the first resolution (the low resolution) in which the voxel size of the first medical image is relatively large by binning the projection data so as to reduce the computational load, i.e., by combining multiple pixels with each other so as to increase a single pixel size.

For example, when the unit pixel size of the projection data acquired by the projection data acquisition unit 110 is 0.1 mm, the first medical image generation unit 120 may reconstruct the first medical image by binning 16 unit pixels each in an x-axis direction and a y-axis direction, i.e., 256 unit pixels (16 X 16), from the projection data. However, the present disclosure is not limited thereto.

Here, for example, binning may involve assigning a representative value to multiple unit pixels by using an average value of multiple unit pixel values or any one unit pixel value, but the present disclosure is not limited thereto.

In addition, as described above, the first medical image may include a three-dimensional image reconstructed on the basis of binned projection data, and may be a full CT image of the entire FOV.

According to an embodiment of the present disclosure, the display unit 130 may display at least one cross-sectional image among a plurality of cross-sectional images of the first medical image on a screen. Specifically, the cross-sectional image is generated through Multi Planar Reformation (MPR), and may be expressed as an MPR cross-sectional image.

Here, the plurality of cross-sectional images may include cross-sectional images for axial, sagittal, and coronal planes. That is, the display unit 130 may display at least one cross-sectional image selected from a cross-sectional image in an axial direction of the first medical image, a cross-sectional image in a sagittal direction of the first medical image, and a cross-sectional image in a coronal direction of the first medical image on the screen.

According to an embodiment of the present disclosure, the display unit 130 may display one image (a first cross-sectional image) selected from the cross-sectional image in the axial direction, the cross-sectional image in the sagittal direction, and the cross-sectional image in the coronal direction on one region of the screen, and may display the other image (a second cross-sectional image) selected from the cross-sectional image in the axial direction, the cross-sectional image in the sagittal direction, and the cross-sectional image in the coronal direction on the other region of the screen. In other words, the display unit 130 may display the first cross-sectional image and the second cross-sectional image among the plurality of cross-sectional images on the screen.

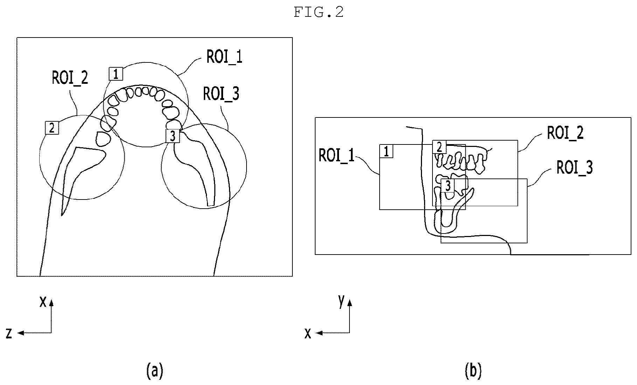

FIG. 2(a) and FIG. 2(b) show an example of the screen in which regions of interest provided by the medical image generation apparatus according to an embodiment of the present disclosure are selected.

Specifically, FIG. 2(a) may illustrate a cross-sectional image in the axial direction, and FIG. 2(b) may illustrate a cross-sectional image in the sagittal direction. In other words, referring to FIG. 2(a) and FIG. 2(b), the display unit 130 may display the cross-sectional image in the axial direction and the cross-sectional image in the sagittal direction on each region of the screen, but the present disclosure is not limited thereto.

Referring to FIG. 2(a) and FIG. 2(b), the region-of-interest selection unit 140 may receive a selection of a region of interest that is a part of the FOV on the basis of the first medical image.

As an example, the region-of-interest selection unit 140 may receive a selection of a partial region in the first medical image displayed on the screen through a user input applied through the input unit. At this time, since the first medical image is an image corresponding to the entire FOV, the partial region selected from the first medical image may be included in the entire FOV, and the partial region may be a region of interest that the user intends to check.

Hereinafter, for the convenience of the description, the cross-sectional image in the axial direction is an image in an xz plane, the cross-sectional image in the sagittal direction is an image in an xy plane, and the cross-sectional image in the coronal direction is an image in a yz plane, but the present disclosure is not limited thereto.

In this regard, in order to designate the region of interest in a CT image constructed in three dimensions, inputs from at least two directions selected from the axial direction, the sagittal direction, and the coronal direction are required. For example, referring to FIG. 2(a) and FIG. 2(b), the region of interest may be a cylindrical shape or a rectangular shape in three dimensions. Specifically, the input in the cross-sectional image in the axial direction is to select a position and a size on the xz plane. Here, the size may be an area (or a horizontal length and a vertical length) of a cross-sectional surface perpendicular to a longitudinal direction of the cylindrical shape or the rectangular shape. **In** addition, the input in the cross-sectional image in the sagittal direction is to select a position and a size on the xy plane. Here, the size may be the vertical length (or the horizontal length) of the cross-sectional surface perpendicular to the longitudinal direction of the cylindrical shape or the rectangular shape and a height of the cylindrical shape or the rectangular shape. In addition, the input in the cross-sectional image in the coronal direction is to select a position and a size on the yz plane. Here, the size may be the horizontal length (or the vertical length) of the cross-sectional surface perpendicular to the longitudinal direction of the cylindrical shape or the rectangular shape and the height of the cylindrical shape or the rectangular shape. Therefore, in order to designate the region of interest in three dimensions, all inputs for the horizontal length, the vertical length, and the height of the cross-sectional surface perpendicular to the longitudinal direction of the cylindrical shape or the rectangular shape are required, so that a region designation on at least two planes selected from the three planes is required to be performed.

Therefore, in order to designate the region of interest in three dimensions, the region-of-interest selection unit 140 may receive both a first selection of the region of interest based on the first cross-sectional image displayed on the screen and a second selection of the region of interest based on the second cross-sectional image.

However, the present disclosure is not limited thereto. Furthermore, when a predetermined value exists for at least one selected from the horizontal length, the vertical length, and the height of the cross-sectional surface perpendicular to the longitudinal direction of the cylindrical shape or the rectangular shape corresponding to the region of interest, the region-of-interest selection unit 140 may determine the region of interest only by designating a region on one of the three planes.

For example, the region-of-interest selection unit 140 may determine the region of interest by receiving the input on the position and the size in the xz plane in the cross-sectional image in the axial direction and the input on the height in the xy plane in the cross-sectional image in the sagittal direction. Furthermore, when the height of the region of interest is already set, the region-of-interest selection unit 140 may determine the region of interest by receiving only the input on the position and the size in the xz plane in the cross-sectional image in the axial direction.

The region-of-interest selection unit 140 may receive at least one selection selected from the first selection of the region of interest based on the first cross-sectional image displayed on the screen and the second selection of the region of interest based on the second cross-sectional image.

Here, the display unit 130 may display the first cross-sectional image and the second cross-sectional image on each region on the screen, but the present disclosure is not limited thereto. Furthermore, the display unit 130 may sequentially display the first cross-sectional image and the second cross-sectional image on the same region on the screen. Furthermore, the region-of-interest selection unit 140 may sequentially receive the first selection of the region of interest based on the first cross-sectional image and the second selection of the region of interest based on the second cross-sectional image.

In addition, the region-of-interest selection unit 140 may receive a selection of a first region of interest and a selection of a second region of interest that are based on one cross-sectional image among the plurality of cross-sectional images. That is, the region-of-interest selection unit 140 may receive selections of a plurality of regions of interest for one cross-sectional image (see ROI_1 to ROI_3 in FIG. 2(a) and FIG. 2(b)).

According to an embodiment of the present disclosure, the second medical image generation unit 150 may generate the second medical image of the region of interest at a second resolution by using at least a portion of the projection data.

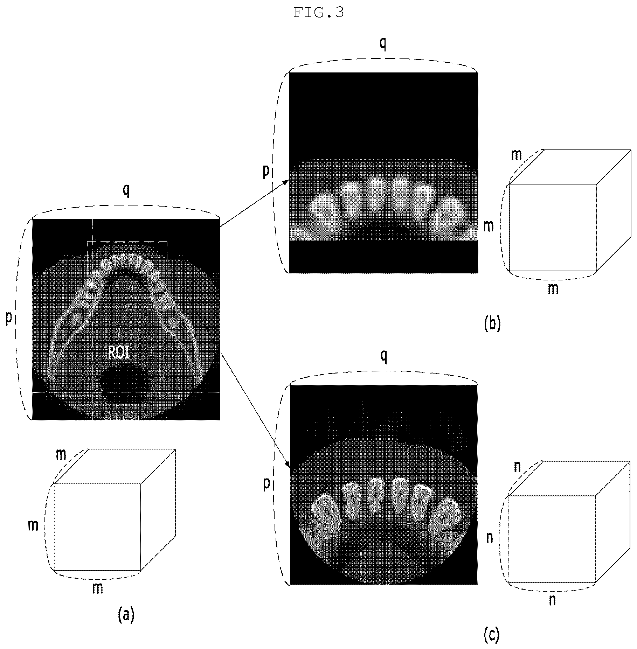

FIG. 3(a), FIG. 3(b), and FIG. 3(c) show a difference in resolution according to voxel sizes of the first medical image and the second medical image provided by the medical image generation apparatus according to an embodiment of the present disclosure.

Specifically, FIG. 3(a) is a view exemplarily illustrating the first medical image and a voxel size mXm for the first medical image. In addition, FIG. 3(b) is a view exemplarily illustrating a comparison image illustrated for a resolution comparison of a region of interest (ROI) when a reconstruction to the second medical image is not performed and a voxel size mXm for the comparison image. In addition, FIG. 3(c) is a view illustrating the reconstructed second medical image of the region of interest ROI and a voxel size nXn for the second medical image. Here, for example, the images in FIG. 3(a), FIG. 3(b), and FIG. 3(c) may be MPR cross-sectional images, but the present disclosure is not limited thereto.

Here, p and q illustrated in FIG. 3(a), FIG. 3(b), and FIG. 3(c) illustrate a size of a screen displayed by the display unit 130, and may be illustrated so as to indicate a resolution difference for the same screen size.

Referring to FIG. 3(a), FIG. 3(b), and FIG. 3(c), the second medical image generation unit 150 may be configured to generate the second medical image of the region of interest ROI by using the projection data corresponding to the region of interest ROI selected through the region-of-interest selection unit 140. Here, the second medical image may be generated such that the second medical image has the second resolution, and the second resolution may refer to a relatively high resolution compared to the first resolution.

In other words, when the voxel size for the first medical image is mXm, the second medical image may be an image generated (reconstructed) at the second resolution with a relatively smaller voxel size (nXn, where m > n) compared to mXm, i.e., the second medical image may be an image generated (reconstructed) at the high resolution. That is, the second medical image may be reconstituted with a voxel size that is relatively small compared to the voxel size of the first medical image described above, so that the number of voxels in the unit volume in the second medical image is relatively larger than the number of voxels in the unit volume in the first medical image.

For example, when the unit pixel size of the projection data acquired by the projection data acquisition unit 110 is 0.1 mm, the second medical image generation unit 150 may reconstruct only a part corresponding to the region of interest ROI in the projection data into the second medical image having a voxel size of 0.07 mm considering a magnification rate. In other words, the second medical image generation unit 150 may reconstruct the projection data of the region of interest into the second medical image having a voxel size that is significantly smaller than the voxel size of the first medical image.

Accordingly, as illustrated in FIG. 3(a), FIG. 3(b), and FIG. 3(c), the resolution of the second medical image that represents the region of interest ROI with the voxel size of nXn smaller than the voxel size of mXm for the first medical image may be significantly increased compared to the resolution of the first medical image and the resolution of the comparison image (see FIG. 3(b)) that represents the region of interest ROI with the voxel size of mXm that is the same as the voxel size of mXm of the first medical image.

In addition, the second medical image generation unit 150 may generate the second medical image by using the projection data, and may reconstruct the projection data into the second medical image by using at least a portion of the projection data corresponding to the region of interest ROI rather than the entire FOV, so that the second medical image of the region of interest at the high resolution may be generated relatively quickly.

In addition, as described above, the second medical image may include a three-dimensional image reconstructed on the basis of the projection data. Furthermore, since the second medical image is an image of the region of interest from the projection data, the second medical image may be a partial CT image corresponding to the region of interest.

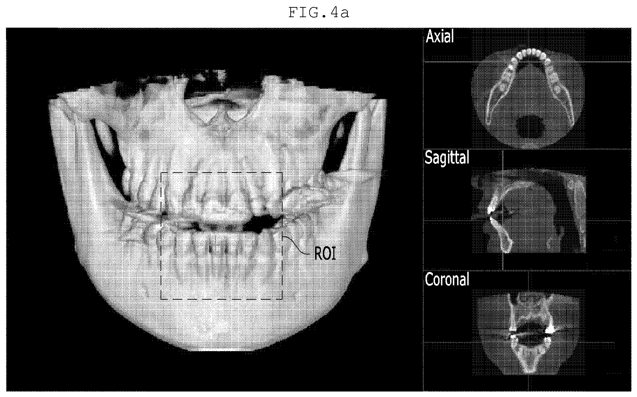

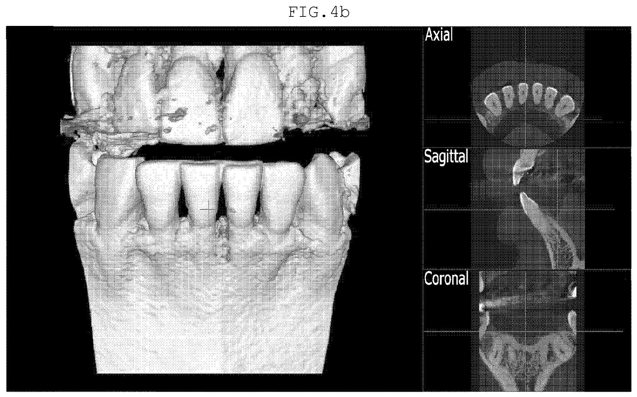

FIG. 4a and FIG. 4b are views illustrating the first medical image expressed in three dimensions and the second medical image expressed in three dimensions that are provided by the medical image generation apparatus according to an embodiment of the present disclosure.

In FIG. 3(a), FIG. 3(b), and FIG. 3(c), the first medical image and the second medical image are illustrated as MPR cross-sectional images. However, as described above, the first medical image and the second medical image may be expressed as three-dimensional images. Specifically, FIG. 4a may illustrate the first medical image configured in three dimensions with respect to the entire FOV, and FIG. 4b may illustrate the second medical image configured in three dimensions with respect to the region of interest (ROI).

Referring to FIG. 4a and FIG. 4b, it can be seen that the resolution of the second medical image (see FIG. 4b) is significantly increased compared to the resolution of the first medical image of the teeth (see FIG. 4a).

In addition, when a plurality of regions of interest is received for any one first medical image, the second medical image generation unit 150 may generate second medical images of each of regions of interest, and may provide the plurality of the generated second medical images as a series formed as one group. In other words, the second medical image generation unit 150 may generate the second medical image of the first region of interest and the second medical image of the second region of interest as a series for the same subject.

In addition, in the second medical image generation unit 150, when a plurality of regions of interest is received for any one first medical image, the second medical image of each of regions of interest may be generated with different resolutions. For example, the second medical image generation unit 150 may generate the second medical image of the first region of interest at the second resolution, and may generate the second medical image of the second region of interest at a third resolution, the third resolution being different from the first resolution and the second resolution described above.

However, the present disclosure is not limited thereto. Furthermore, the second medical image generation unit 150 may not generate a plurality of second medical images of each of the plurality of regions of interest but may generate one second medical image in which the projection data corresponding to the plurality of regions of interest is reconstructed at the second resolution. Otherwise, the second medical image generation unit 150 may generate the second medical images such that at least one selected from the plurality of regions of interest has the second resolution and at least one of the other selected from the plurality of regions of interest has the third resolution.

In addition, the second medical image generation unit 150 may generate the second medical image of the region of interest at the second resolution based on at least one cross-sectional image and having a fourth resolution for a surrounding region of the region of interest based on at least one cross-sectional image, the fourth resolution being different from the first resolution and the second resolution.

Specifically, the second medical image generation unit 150 may generate the second medical image such that the region of interest is reconstructed at the second resolution and a part corresponding to a surrounding region in the projection data is reconstructed at the fourth resolution, the surrounding region being a region from a boundary of the region of interest to a boundary of an enlarged region in which the region of interest is enlarged with a predetermined rate on the basis of the region of interest received from the region-of-interest selection unit 140. In other words, the second medical image generation unit 150 may be configured to generate the second medical image by reconstructing the region of interest in the projection data at the second resolution and reconstructing the surrounding region at the fourth resolution.

Here, the fourth resolution may be a resolution which is higher than the first resolution and which is smaller than the second resolution, and may be the same as the third resolution, but the present disclosure is not limited thereto. Furthermore, the third resolution may be the same as the fourth resolution.

According to an embodiment of the present disclosure, the display unit 130 may display the second medical image of the region of interest based on a displayed cross-sectional image on the screen. Specifically, the display unit 130 may display at least one cross-sectional image among the plurality of cross-sectional images of the first medical image, and may display the second medical image of the received region of interest when a selection of the region of interest based on the displayed cross-sectional image is received. In other words, the region of interest based on the first cross-sectional image and the second medical image of the region of interest based on the second cross-sectional image may be displayed on the screen.

Here, the display unit 130 may display the second medical image on a region other than the region in which the first medical image is displayed on the screen, but the present disclosure is not limited thereto. Furthermore, the display unit 130 may display the second medical image by overlapping the second medical image with the first medical image.

In addition, the display unit 130 may display a selection of the received region of interest by overlapping the selection with the first medical image or the second medical image such that the selection is capable of being identified.

As described above, the medical image generation apparatus 100 according to an embodiment of the present disclosure generates a three-dimensional medical image of the entire FOV at a relatively low resolution, and generates a three-dimensional medical image of the region of interest selected by the user input at a high resolution, so that the region of interest may be displayed with the high resolution rapidly.

Hereinafter, an operation flow of the present disclosure will be briefly described on the basis of the detailed description described above.

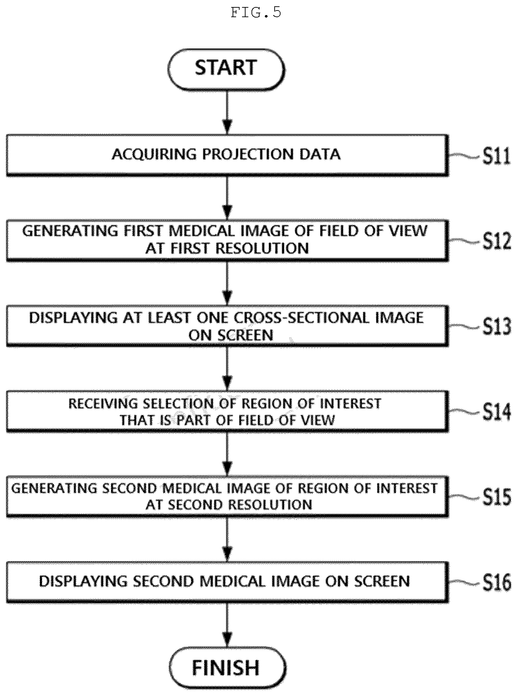

FIG. 5 is an operation flowchart illustrating a medical image generation method according to an embodiment of the present disclosure.

The medical image generation method illustrated in FIG. 5 may be performed by the medical image generation apparatus 100 described above. Therefore, even when a description is omitted hereinafter, the description of the medical image generation apparatus 100 may be equally applied to the description of the medical image generation method.

Referring to FIG. 5, in a process S11, the projection data acquisition unit 110 may acquire projection data of a FOV.

Next, in a process S12, the first medical image generation unit 120 may generate a first medical image of the FOV at a first resolution.

Next, in a process S13, the display unit 130 may display at least one cross-sectional image of the first medical image on the screen.

Next, in a process S14, the region-of-interest selection unit 140 may receive a selection of a region of interest that is a part of the FOV on the basis of the first medical image. In the process S14, the region-of-interest selection unit 140 may receive the selection of the region of interest on the basis of at least one cross-sectional image displayed on the screen.

Next, in a process S15, the second medical image generation unit 150 may generate a second medical image of the region of interest at a second resolution by using the projection data, the second resolution being higher than the first resolution.

Next, in a process S16, the display unit 130 may display the second medical image of the region of interest on the screen. Specifically, in the process S16, the display unit 130 may display the region of interest based on a first cross-sectional image and the second medical image of the region of interest based on a second cross-sectional image on the screen.

Here, the first medical image and the second medical image are formed of voxels, the first resolution and the second resolution may correspond to the number of voxels in the unit volume, and the second medical image may have a relatively large number of voxels in the unit volume compared to that of the first medical image.

In addition, the first medical image and the second medical image may be three-dimensional images, and may be CT images. Specifically, the first medical image may be a full CT image of the FOV, the second medical image may be a partial CT image corresponding to the region of interest, and at least one cross-sectional image may be any one selected from a cross-sectional image in the axial direction and a cross-sectional image in the sagittal direction.

In the description described above, the process S11 to the process S16 may be further divided into additional processes or may be combined into fewer processes according to an embodiment of the present disclosure. In addition, some processes may be omitted as required, and the order between the processes may be changed.

The medical image generation method according to an embodiment of the present disclosure may be implemented as a program instruction executable by a variety of computer mechanisms and may be recorded on a computer-readable medium. The computer-readable medium may include a program instruction, a data file, a data structure, and so on alone or in combination. The program instruction recorded on the medium may be designed and configured specifically for an embodiment of the present disclosure or may be publicly known and available to those who are skilled in the field of computer software. Examples of the computer-readable recording medium include a magnetic medium, such as a hard disk, a floppy disk, and a magnetic tape, an optical medium, such as a compact disk read-only memory (CD-ROM), a digital versatile disc (DVD), and so on, a magneto-optical medium such as a floptical disk, and a hardware device specially configured to store and perform the program instruction, such as a read-only memory (ROM), a random access memory (RAM), a flash memory, and so on. Examples of the computer instruction include not only machine language code generated by a compiler, but also include high-level language code executable by a computer using an interpreter and so on. The hardware device may be configured to operate as one or more software modules in order to perform the operation of the present disclosure, and vice versa.

Furthermore, the medical image generation method described above may be implemented in a form of a computer program or an application executed by the computer, the computer program or the application being stored in the recording medium.

The aforementioned description of the present disclosure is used for exemplification, and it can be understood by those skilled in the art that the present disclosure can be easily modified in other detailed forms without changing the technical spirit or requisite features of the present disclosure. Therefore, it should be understood that the embodiments described above are illustrative in all aspects and not restrictive. For example, respective constituent elements described as single types can be distributed and implemented and, similarly, constituent elements described to be distributed can also be implemented in a coupled form.

The scope of the present disclosure is represented by claims to be described below rather than the detailed description, and it is to be interpreted that the meaning and scope of the claims and all the changes or modified forms derived from the equivalents thereof come within the scope of the present disclosure.

## Claims

1. A medical image generation apparatus comprising:
a projection data acquisition unit configured to acquire projection data of a field of view;
a first medical image generation unit configured to generate a first medical image of the field of view at a first resolution by using the projection data;
a region-of-interest selection unit configured to receive a selection of a region of interest that is a part of the field of view on the basis of the first medical image; and
a second medical image generation unit configured to generate a second medical image of the region of interest at a second resolution by using the projection data, the second resolution being higher than the first resolution.

2. The medical image generation apparatus of claim 1, wherein the first medical image and the second medical image are formed of voxels, the first resolution and the second resolution correspond to a number of voxels in unit volume, and the number of voxels in unit volume of the second medical image is relatively greater than the number of voxels in unit volume of the first medical image.

3. The medical image generation apparatus of claim 1, wherein the first medical image and the second medical image are three-dimensional images,
wherein the medical image generation apparatus further comprises a display unit configured to display at least one cross-sectional image of the first medical image on a screen, and
wherein the region-of-interest selection unit is configured to receive the selection of the region of interest on the basis of the at least one cross-sectional image displayed on the screen.

4. The medical image generation apparatus of claim 1, wherein the first medical image and the second medical image are Computed Tomography (CT) images, the first medical image is a full CT image of the field of view, and the second medical image is a partial CT image corresponding to the region of interest.

5. The medical image generation apparatus of claim 3, wherein the first medical image and the second medical image are Computed Tomography (CT) images, and the at least one cross-sectional image is at least one selected from a cross-sectional image in an axial direction and a cross-sectional image in a sagittal direction.

6. A medical image generation method comprising:
(a) acquiring projection data corresponding to a field of view;
(b) generating a first medical image of the field of view at a first resolution by using the projection data;
(c) receiving a selection of a region of interest that is a part of the field of view on the basis of the first medical image; and
(d) generating a second medical image of the region of interest at a second resolution using the projection data, the second resolution being higher than the first resolution.

7. The medical image generation method of claim 6, wherein the process (c) further comprises displaying at least one cross-sectional image of the first medical image on a screen, and
wherein the selection of the region of interest is received on the basis of the at least one cross-sectional image.
